# EUROPÄISCHE PATENTSCHRIFT

(11) **EP 0 445 156 B1**
(45) Veröffentlichungstag und Bekanntmachung des Hinweises auf die Patenterteilung: **10.02.1993**
(21) Anmeldenummer: 89912949.8
(22) Anmeldetag: 24.11.1989
(51) Int. Cl.: A61K 31/505, A61K 9/36, A61K 9/10

(54) **MITTEL ZUR BEHANDLUNG VON CHRONISCH ENTZÜNDLICHEN DARMERKRANKUNGEN**
AGENTS FOR TREATING CHRONIC INFLAMMATORY DISEASES OF THE INTESTINES
MEDICAMENTS POUR LE TRAITEMENT DE MALADIES INFLAMMATOIRES CHRONIQUES DES INTESTINS

(30) Priorität: 25.11.1988 DE 3839839
(43) Veröffentlichungstag der Anmeldung: 11.09.1991
(73) Patentinhaber: HENNING BERLIN GmbH CHEMIE- UND PHARMAWERK, D-12099 Berlin (DE)
(72) Erfinder: SCHEIFFELE, Ekkehard, D-1000 Berlin 46 (DE); GERBER, Gerhard, DDR-1136 Berlin (DD); SIEMS, Werner, DDR-1136 Berlin (DD); WERNER, Andreas, DDR-1152 Berlin (DD)
(74) Vertreter: Werner, Hans-Karsten, Dr.Dipl.-Chem.
(86) Internationale Anmeldenummer: EP8901426
(87) Internationale Veröffentlichungsnummer: WO9006116

(56) Entgegenhaltungen:
- EP-A- 0 062 000
- EP-A- 0 261 439
- AT-A- 352 686
- GB-A- 975 850

## Beschreibung

Chronisch-entzündliche Darmerkrankungen wie Morbus Crohn und Colitis ulcerosa sind schwere, das Befinden der betroffenen Patienten sehr beeinträchtigende Krankheiten, die nicht selten zur Berufsunfähigkeit führen und unter Umständen radikale chirurgische Maßnahmen mit den entsprechenden Folgeerscheinungen erforderlich machen.

Ein kausaltherapeutisches Prinzip steht bislang noch nicht zur Verfügung. Die Behandlung erfolgt mit Sulfasalazin oder Mesalazin und ist meist unbefriedigend. Häufig ist der Einsatz von Corticoiden hochdosiert erforderlich. Bei der Pathogenese und der Unterhaltung der chronisch-entzündlichen Darmerkrankungen spielen nach neueren Erkenntnissen sogenannte Superoxid-Radikale (O₂-Radikale) und die durch sie ausgelöste Lipidperoxydation eine maßgebliche Rolle. Durch Hemmung ihrer Entstehung wäre eine wirksame Behandlung der Erkrankungen gegeben.

Ein wesentlicher Anteil der im Darm entstehenden Superoxid-Radikale werden im Verlauf der Purin-Oxidation (Bildung von Harnsäure aus Hypoxanthin und Xanthin) durch die Wirkung der Darm-Xanthinoxidase gebildet. Das läßt sich u.a. an dem Abfall der Adenin- und Guaninnukleotidkonzentrationen zeigen.

Allopurinol (ein Hemmer der Xanthinoxidase) ist seit über 20 Jahren ein Standardtherapeutikum zur Behandlung der Hyperurikämie und der Gicht. Es wird im Organismus in erheblichem Umfang in Oxipurinol umgewandelt, das als Hauptmetabolit für die Langzeitwirkung von Allopurinol verantwortlich ist. Gelegentlich wurde auch schon Oxipurinol, das selbst jedoch schlecht resorbierbar ist, zur Behandlung der Gicht eingesetzt. Es wurde jetzt gefunden, daß überraschenderweise Oxipurinol und/oder seine Alkali-, Erdalkali- oder Ammoniumsalze bei enteraler Applikation geeignet sind, chronisch entzündliche Darmerkrankungen wie Morbus Crohn und Colitis ulcerosa effektiv zu behandeln. Dazu ist es allerdings nötig, diese Wirkstoffe so zuzubereiten, daß sie im mittleren und/oder unteren Bereich des Dünndarms zur Verfügung stehen. Dies bedeutet, daß orale Applikationen magensaftresistent sind und dadurch erst im mittleren und/oder unteren Bereich des Dünndarms freigesetzt werden. Es besteht aber auch die Möglichkeit, diese Wirkstoffe rektal in Form von Klysmen zur Anwendung zu bringen.

Oxipurinol ist sehr schwer löslich und kann daher nicht einmal in mikronisieiter Form ausreichend resorbiert werden, um als Mittel zur Behandlung der Hyperurikämie und der Gicht angewendet zu werden. Es wurde von der Anmelderin in anderem Zusammenhang festgestellt, daß die bisher noch nicht beschriebenen Alkali- und/oder Erdalkalisalze des Oxipurinols in amorpher oder kristalliner Form offensichtlich bereits im Magen und im oberen Bereich des Dünndarms resorbiert werden und daher auch zur Behandlung der Hyperurikämie und Gicht verwendet werden können.

Aus der DE-OS 37 07 999 ist bekannt, Oxipurinol zur Verminderung von Zellschäden nach der Ischämie und vor der Reperfusion zu verwenden, und zwar durch intravenöse Verabreichung eines Alkalimetallsalzes, insbesondere des Natriumsalzes. Aus den Beispielen geht jedoch hervor, daß nicht das reine Natriumsalz verwendet wurde, sondern ein Gemisch aus Salz und überschüssiger Natronlauge. Dies ergibt sich vor allem aus den pH-werten der ca. 1 bis 2%-igen Lösungen von 11,5 bzw. 12,0. Das jetzt erstmals von der Anmelderin in reiner und kristalliner Form hergestellte Natriumsalz besitzt in 5%-iger wäßriger Lösung einen pH-wert von 9,7 und kristallisiert als Monohydrat aus.

Die GB-A-975,850 beschreibt Xanthinoxydaseinhibitoren, wobei als aktive Verbindungen 4-hydroxy-pyrazolo[3,4-d]pyrimidin und 4,6-dihydroxypyrazolo[3,4-d]pyrimidin beschrieben werden. Dort wird beschrieben, daß diese Verbindungen sich durch geringe Toxizität, geringe Nebenwirkungen und lange Verweilzeiten ohne Eliminierung und Ausscheidung oder metabolischen Abbau auszeichnen. Die Verbindungen hatten die Wirkung, daß nach Verabreichung dieser Arzneimittel der Gehalt von Urinsäuren im Blut und im Urin signifikant zurückgingen und statt dessen eine erhöhte Ausscheidung von Hyoxanthin und Xanthin erfolgte. Daraus ergibt sich die Eignung dieser Verbindung als Therapeutika zur Behandlung von Gicht.

Die EP-A-0,062,000 beschreibt die Wirkung von 4-Aminosalicylsäure als Mittel zur Behandlung von Entzündungen.

Die AT-A-352,686 beschreibt die Mikroeinkapselung von verschiedenen Arzneimittel, unter anderem auch Allopurinol. Dabei wird Allopurinol homogenisiert, dispergiert in Gegenwart von Dimethylolharnstoff und Gelatine. Die Kaspelsuspensionsschicht wird gewonnen durch Dispergierung dieser Mischung bei 40°C, einer mittleren Drehzahl von 5.000 U/m und Einstellen des pH-Wertes auf 4, woraufhin 30 Gew.-%ige wäßrige Natriumsulfatlösung hinzugegeben wird und mit Eiswasser auf 8°C abgekühlt wird. Danach wird eine 30 Gew.-%ige Gerbsäurelösung hinzugefügt.

Die EP-A-0,261,439 beschreibt ein Arzneimittel zur Behandlung von Ateriosclerose enthaltend Pyrimido[2,1-b]benzothiazolederivate. Diese Verbindungen werden dort als blutfettgehaltreduzierende Mittel beschrieben. Des weiteren wird als pharmakologische Wirkung eine Thrombozytenaggregationshemmung beschrieben.

Gegenstand der vorliegenden Erfindung sind somit Mittel zur enteralen Behandlung von chronisch entzündlichen Darmerkrankungen enthaltend neben üblichen Träger- und Hilfsstoffen pharmakologisch wirksame Dosen von Oxipurinol und/oder seine Alkali-, Erdalkali- oder Ammoniumsalze , die den Wirkstoff erst im mittleren und/oder unteren Bereich des Dünndarms freisetzen oder als Klysma einsetzbar sind.

Ein bevorzugter Gegenstand der vorliegenden Erfindung sind Mittel, die dadurch gekennzeichnet sind, daß sie für die orale Applikation geeignet sind, magensaftresistent sind und erst im mittleren und/oder unteren Bereich des Dünndarms freigesetzt werden. Vorzugsweise sind diese Mittel dadurch gekennzeichnet, daß sie das Oxipurinol in Form von Alkali- und/oder Erdalkalisalzen in amorpher oder kristalliner Form enthalten und als magensaftresistente Tabletten oder Kapseln vorliegen.

Eine bevorzugte Ausführungsform der Erfindung sind Mittel, welche das Oxipurinol und/oder seine Alkali-, Erdalkali- oder Ammoniumsalze suspendiert oder gelöst enthalten und in Form von magensaftresistenten Kapseln vorliegen.

Ein weiterer Gegenstand der Erfindung sind Mittel, welche das Oxipurinol und/oder seine Alkali-, Erdalkalioder Ammoniumsalze in fester, suspendierter oder gelöster Form enthalten und in Form von Klysmen vorliegen.

Die erfindungsgemäßen, oral anwendbaren Mittel enthalten im allgemeinen 50 bis 800 mg Wirkstoff pro Dosiseinheit. Klysmen enthalten im allgemeinen 50 bis 500 mg Wirkstoff pro Dosiseinheit.

Obwohl es für die Behandlung von chronisch entzündlichen Darmerkrankungen darauf ankommt, daß das Oxipurinol im Darm verbleibt, sind dennoch Mittel gut geeignet, welche das Oxipurinol im entzündeten Darmbereich, d.h. im unteren Bereich des Dünndarms bzw. im Dickdarm zur Lösung gelangen lassen, um einen guten Kontakt mit der Mucosa zu gewährleisten. Hierzu muß der Wirkstoff in leichtlöslicher Form bzw. Kolloid oder dispers vorliegen. Bei solchen Zubereitungen ist darauf zu achten, daß der Wirkstoff magensaftresistent zubereitet ist und erst im mittleren oder unteren Bereich des Dünndarms freigesetzt wird. Selbstverständlich ist es auch möglich, derartige Zubereitungen rektal in Form von Klysmen zur Anwendung zu bringen.

Erfindungsgemäß ist es weiterhin möglich, Oxipurinol und/oder seine Alkali-, Erdalkali-'oder Ammoniumsalze zusammen mit den bisher verwendeten Mitteln Sulfasalazin oder Mesalazin zu kombinieren und zur Anwendung zu bringen.

Magensaftresistente Zubereitungen weisen im allgemeinen einen Überzug auf, der sich erst oberhalb eines pH-Wertes von 3, vorzugeweiee erst im pH-Bereich von 5 bis 7,5 löst und den Wirkstoff erst in dem Bereich des Verdauungstraktes freisetzt, in dem entsprechend hohe pH-Werte vorliegen. Als Überzüge eignen sich beispielsweise bekannte Überzugsmittel wie Polymere der Acrylsäure (Eudragite^{R}), insbesondere deren wäßrig dispergierbare Formen und Mischungen, sowie Cellulosederivate wie Hydroxypropylmethylcellulose und deren Derivate, nämlich Phtalate, Acetatsuccinate oder Celluloseacetatphtalate. Aus diesen Zubereitungsformen wird das Oxipurinol erst im unteren Bereich des Dünndarms und im Dickdarm zur Verfügung gestellt und bewirkt dort eine effektive Hemmung der Xanthin-Oxidase und unterbindet damit die Entstehung der als Entzündungsmediatoren fungierenden Superoxid-Radikale. Es hat sich gezeigt, daß Oxipurinol und/oder seine Alkali-, Erdalkali- oder Ammoniumsalze bei der Behandlung von chronisch entzündlichen Darmerkrankungen sowohl in der akuten Phase als auch zur Verhütung eines Rezidivs geeignet sind.

Die pharmakologisch wirksamen Dosen betragen bei oraler Applikation im allgemeinen 100 bis 800 mg pro Tag und 50 bis 500 mg pro Tag bei rektaler Applikation.

Die Herstellung der neuen Alkali- und Erdalkalisalze des Oxipurinols in amorpher oder kristalliner Form erfolgt in an sich bekannter weise. Da sie gegenüber mikronisiertem Oxipurinol besser und schneller löslich sind, stellen sie zwar den Wirkstoff rascher und in größeren Mengen zur Verfügung, gleichzeitig führt dies aber auch zu erhöhter Resorption des Wirkstoffes in das Serum. Dies kann erwünscht sein bei Patienten mit Hyperurikämie und Gicht. Durch die spezielle galenische Zubereitung gelingt es jedoch, die Resorptionsquote zu begrenzen. Eine geringe Resorption des Wirkstoffes durch die Darmwand stört nicht, da Oxipurinol in der vorgesehenen Dosis gut verträglich ist und gut renal eliminiert wird. Entscheidend für die Behandlung der chronisch entzündlichen Darmerkrankungen ist, daß eine ausreichende Menge an Oxipurinol im Darm zur Verfügung steht, um die Xanthinoxidase effektiv zu hemmen und damit die Entstehung der als Entzündungsmediatoren fungierenden Superoxid-Radikale zu unterbinden.

Neben den erfindungsgemäßen Mitteln ist ein weiterer Gegenstand der vorliegenden Erfindung die Verwendung von Oxipurinol und/oder seiner Alkali-, Erdalkali- oder Ammoniumsalze zur Herstellung von Mitteln zur enteralen Behandlung von chronisch entzündlichen Darmerkrankungen.

Schließlich ist Gegenstand der Erfindung ein Verfahren zur enteralen Behandlung von chronisch entzündlichen Darmerkrankungen durch Applikation von pharmakologisch wirksamen Dosen des Oxipurinols und/oder seiner Alkali-, Erdalkali- und/oder Ammoniumsalze.

In den nachfolgenden Beispielen sind die verschiedenen Gegenstände dieser Erfindung näher erläutert.

### Beispiel 1

### Herstellung von Oxipurinol-Natrium

1 Gewichtsteil reines Oxipurinol wird mit 16,5 bis 17 Gewichtsteilen entionisiertem Wasser aufgeschlämmt. Unter Rühren und Aufheizen bis 90°C wird ab 40°C 30%-ige reine Natronlauge jeweils bis pH 11 zugegeben und heiß filtriert. Durch schnelles Abkühlen auf ca. 25°C bildet sich ein Kristallbrei, der weiter bis auf 1 bis 2°C gekühlt wird. Der Kristallbrei wird abgetrennt und mehrmals mit reinem Methanol gewaschen und bei 60°C getrocknet.

Ausbeute ca. 80% Oxipurinol-Natrium.

Die Aufarbeitung der Mutterlauge liefert weitere 15%.

### Eigenschaften von Oxipurinol-Natrium

Summenformel: C₅H₃N₄O₂Na · H₂O
MG. 192,1

Bei diesem Natrium-Salz handelt es sich um Mono-Natriumoxipurinol-Monohydrat. Das Salz ist weiß und kristallisiert in feinen Nadeln. In wäßriger Lösung reagiert es alkalisch, eine 5%ige Lösung besitzt einen pH-Wert von 9,7. Seine Löslichkeit beträgt:
in Wasser bei
25°C 15,8 g/l
0°C 7,2 g/l
in Methanol/Wasser (3:1 Vol.) bei 25°C ca. 2,0 g/l

Das Salz ist luftbeständig und verträgt Trockentemperaturen bis etwa 70°C. Aus seiner heißen Lösung läßt sich mit verdünnter Salzsäure (10%ig) Oxipurinol fast quantitativ ausfällen.

### Beispiel 2

### Herstellung von Oxipurinol-Natrium

In einer Mischung aus 25 l reinem Methanol und 2,6 l entionisiertem Wasser wird 1 kg trockenes Oxipurinol suspendiert und innerhalb 1 Stunde eine Lösung von 525 g NaOH, rein, und 4,725 l entionisiertem Wasser unter Rühren zugegeben. Es bildet sich ein Kristallbrei, der nach 3 Stunden Rührzeit abgetrennt (Nutsche bzw. Drucknutsche), portionsweise mit 10 l reinem Methanol gewaschen und bei 60°C getrocknet wird.

Ausbeute ca. 95% Oxipurinol-Natrium.

### Beispiel 3

### Herstellung von Oxipurinol-Kalium

Wie in Beispiel 2 wird Oxipurinol in 90% Methanol suspendiert und entsprechend mit KOH umgesetzt.

Ausbeute ca. 85% Oxipurinol-Kalium.

### Beispiel 4

### Herstellung von Oxipurinol-Magnesium

Die wäßrige Aufschlämmung von reinem Oxipurinol (2 Mol) mit 1 Mol Magnesium-Chlorid wird bei ca. 60°C unter Rühren mit einem Überschub an stark basischem Ionenaustauscherharz (z.B. DOWEX 1 SBR oder Amberlite IRA 400) ungesetzt, das Harz abgetrennt und nach Einengen das Magnesiumsalz mit Isopropanol ausgefällt. Das Magnesiumsalz fällt hierbei amorph an.

### Beispiel 5

### Herstellung von dünndarmlöslichen Oxipurinol-Natrium-Tabletten

113,7 g Oxipurinol-Natrium gemäß Beispiel 1 werden mit 30 g mikrokristalliner Cellulose, 6,0 g quervernetztem Polyvinylpyrrolidon sowie 2,1 g Polyvinylpyrrolidon (mittl. Mol.-Gew. ca. 25000) trocken vermischt, mit Wasser angefeuchtet, das entstandene Granulat getrocknet und anschließend mit 1,2 g Magnesiumstearat vermischt. Aus dem Granulat werden Tabletten mit einem Durchmesser von 3 mm und einer Höhe von 1,6 mm gepreßt, die mit Hydroxypropylmethylcellulose vorisoliert und anschließend mit einem magensaftresistenten Überzug aus wäßrig dispergierbarem Hydroxypropylenmethylcelluloseacetatsuccinat überzogen werden. Der Film löst sich bei einem pH-Wert ab 7,0 schnell und erlaubt eine rasche Freisetzung des Wirkstoffes aus der Darreichungform.

Die Filmtabletten können in Hartgelatinekapseln abgefüllt werden. Es ist auch möglich, das Granulat in Hartgelatinekapseln abzufüllen und die fertigen Kapseln magensaftresistent zu überziehen.

### Beispiel 6

### Herstellung von dünndarmlöslichen Oxipurinol-Natrium-Tabletten

Aus einem Tabletten-Granulat gemäß Beispiel 5 werden Tabletten mit einem Gewicht von 336 mg und einem Gehalt von 250 mg Oxipurinol-Natrium gepreßt. Selbstverständlich können auch Tabletten niedriger oder höherer Dosierungen aus diesem Granulat hergestellt werden. Nach-Vorisolierung gemäß Beispiel 5 werden sie mit einem magensaftresistenten Überzug aus wäßrig dispergierbarem Hydroxypropylenmethylcelluloseacetatsuccinat überzogen.

Der Film löst sich bei einem pH-wert ab 7,0 schnell und erlaubt eine rasche Freisetzung des Wirkstoffes aus der Darreichungsform.

### Beispiel 7

### Herstellung von dünndarmlöslichen Oxipurinol-Natrium-Tabletten mit verzögerter Wirkstoff-Freisetzung

Aus einem Tabletten-Granulat gemäß Beispiel 5 werden Tabletten mit einem Durchmesser von 2 mm und einer Höhe von 1,2 mm gepreßt und nach entsprechender Vorisolierung in an sich bekannter Weise mit einem Film aus Eudragite ^{R}, insbesondere deren wäßrig dispergierbare Form und Mischungen davon, oder einem ähnlichen Filmlack, der eine verzögerte Wirkstoff-Freisetzung im Verlauf der Darmpassage bewirkt, versehen. Anschließend erfolgt die Aufbringung einer magensaftresistenten Filmschicht gemäß Beispiel 5. Die Mini-Tabletten werden zur Formulierung der benötigten Dosisformen in entsprechender Menge in Hartgelatinekapseln gefüllt.

### Beispiel 8

### Herstellung von dünndarmlöslichen Oxipurinol-Tabletten mit verzögerter Wirkstoff-Freisetzung

152,1 g Oxipurinol werden in einer Luftstrahlmühle mikronisiert und gemäß Beispiel 7 zu Hartgelatinekapseln weiterverarbeitet.

### Beispiel 9

### Herstellung von dünndarmlöslichen Oxipurinol-Kalium-Tabletten mit verzögerter Wirkstoff-Freisetzung

Unter Verwendung von Oxipurinol-Kalium statt des Natriumsalzes wird ein Granulat gemäß Beispiel 5 hergestellt. Daraus lassen sich Tabletten in therapiegerechter Dosis, vorzugsweise 60 bis 340 mg pro Tablette, pressen. Die Tabletten werden mit einem Überzug, der eine verzögerte Freisetzung im Darm bewirkt, und zusätzlich mit einem Überzug, der beständig gegen Magensaft ist, versehen.

### Beispiel 10

### Herstellung von Oxipurinol-Natrium-Klysmen

Mikronisiertes Oxipurinol-Natrium wird in einer 0,1 bis 3%igen Natriumcarboxymethylcelluloselösung unter Zusatz üblicher Konservierungsmittel wie Parahydröxybenzoesäureester und geringer Mengen Entschäumer, vorzugsweise Silikonentschäumer, in einer wahlweise einzustellenden Konzentration, vorzugsweise 50 bis 500 mg pro 100 ml Volumen suspendiert und in Klysmenflaschen abgefüllt.

### Beispiel 11

### Herstellung von Oxipurinol-Klysmen

Mikronisiertes Oxipurinol-Natrium wird gemäß Beispiel 10 zur Herstellung von Klysmen verwendet.

### Beispiel 12

### Histologischer Nachweis des entzündungshemmenden Effektes von Oxipurinol-Natrium bei der durch TNB (Trinitrobenzoesulfonsäure) ausgelösten Colitis der Ratte

Bei 10 Ratten (Körpermaße: 200-250 g) wurde durch rektale Instillation von 25 mg TNB (in 0,25 ml 30%iger ethanolischer Lösung) eine Colitis ausgelöst. Eine in gleicher Weise behandelte Gruppe von 10 Tieren erhielt vom 3. - 21. Tage nach der TNB-Gabe im Abstand von 8 Stunden 5 mg Oxipurinol-Na, das in 0,5 ml Glucoselösung aufgelöst war, oral über eine Schlundsonde verabreicht. Eine weitere Gruppe von 10 Tieren erhielt lediglich 0,25 ml der 30%igen Ethanollösung rektal instilliert, und weitere 10 Tiere dienten als Kontrolle.

Der Darm (Rectum) der mit TNB behandelten Tiere zeigte eine chronisch-rezidivierende Colitis Grad II - III. Bei einigen Tieren wurden inkomplette Erosionen, insbesondere in den über den Lymphfollikeln gelegenen Stellen des Oberflächenepithels, sehr dichte Infiltrate in der Submukosa und reichlich perivaskulär gelegene Mastzellen im umgebenden Fettgewebe, beobachtet. Die mit TNB und Oxipurinol-Na behandelten Tiere zeigten deutlich geringer ausgeprägte Entzündungemerkmale (Grad I): eine geringfügige Vermehrung von Lymphozyten und Mastzellen sowie ein geringes Ödem.

### Beispiel 13

### Biochemischer Nachweis des entzündungshemmenden Effektes von Oxipurinol-Na bei der durch TNB (Trinitrobenzoesulfonsäure) ausgelösten Colitis der Ratte

Bei den im Beispiel 13 charakterisierten Tiergruppen wurden am 21. Tag Darmproben auf den Gehalt an Purinnukleotiden und Lipidperoxydationsprodukten (Thiobarbitursäure-reaktive Substanzen: TBA-RS) hin untersucht. Die Gehalte an Adenin- und Guaninnukleotiden der TNB-Oxipurinol-, Ethanol- und Kontrollgruppe unterschieden sich nicht signifikant voneinarider; demgegenüber waren in der TNB-Gruppe das ATP auf 61 %, das GTP auf 40 % und die Summe der Adeninnukleotide auf 71 % abgefallen.

Die nach TNB-Applikation erhöhte Konzentration von TBA-RS wird durch Oxipurinol-Na-Zugabe auf46 % gesenkt und liegt damit sogar noch unter den Kontrollwerten. Danach verhindert Oxipurinol-Na den durch TNB ausgelösten Abfall der Adenin-'und Guaninnukleotide und die gesteigerte Lipidperoxydation.

## Patentansprüche

1. Mittel zur enteralen Behandlung von chronisch entzündlichen Darmerkrankungen enthaltend neben üblichen Trä-ger- und Hilfsstoffen pharmakologisch wirksame Dosen -von Oxipurinol und/oder seine Alkali-, Erdalakli- oder Ammoniumsalze, die den Wirkstoff erst im mittleren und/oder unteren Bereich des Dünndarms freisetzen oder als Klysma einsetzbar sind.

2. Mittel gemäß Anspruch 1, dadurch gekennzeichnet, daß sie für die orale Applikation geeignet sind, magensaftresistent sind und erst im mittleren und/oder unteren Bereich des Dünndarms freigesetzt werden.

3. Mittel gemäß Anspruch 2, dadurch gekennzeichnet, daß sie das Oxipurinol in Form von Alkali- und/oder Erdalkalisalzen in amorpher oder kristalliner Form enthalten und als magensaftresistente Tabletten oder Kapseln vorliegen.

4. Mittel gemäß Anspruch 2, dadurch gekennzeichnet, daß sie das Oxipurinol und/oder seine Alkali-, Erdalkalioder Ammoniumsalze suspendiert oder gelöst enthalten und in Form von magensaftresistenten Kapseln vorliegen.

5. Mittel gemäß Anspruch 1, dadurch gekennzeichnet, daß sie das Oxipurinol und/oder seine Alkali-, Erdalkali- oder Ammoniumsalze In fester, suspendierter oder gelöster Form enthalten und in Form von Klysmen vorliegen.

6. Mittel gemäß einem der Ansprüche 1 bis 4, dadurch gekennzeichnet, daß sie 50 bis 800 mg pro Dosiseinheit enthalten.

7. Mittel gemäß Anspruch 5, dadurch gekennzeichnet, daß sie 50 bis 500 mg pro Dosiseinheit enthalten.

8. Mittel gemäß einem der Ansprüche 1 bis 7, dadurch gekennzeichnet, daß sie zusätzlich Sulfasalazin oder Mesalazin enthalten.

9. Verwendung von Oxipurinol und/oder seiner Alkali-, Erdalkali- oder Ammoniumsalze zur Herstellung von Mitteln zur enteralen Behandlung von chronisch entzündlichen Darmerkrankungen.

10. Verwendung von Oxipurinol und/oder seiner Alkali-, Erdalkali- oder Ammoniumsalze zur Herstellung von Mitteln zur enteralen Behandlung von chronisch entzündlichen Darmerkrankungen, wobei die Mittel eine pharmakologisch wirksame Dosis des Mittels nach mindestens einem der Ansprüche 1 bis 8 enthalten.

## Claims

1. Agents for enteral treatment of chronically inflammatory intestinal diseases, among usual carriers and adjuvants containing pharmacologically active doses of oxypurinol and/or its alkali, alkaline earth, or ammonium salts, which release the active substance only in the mid and/or lower region of the small intestine, or may be used as a clysma.

2. Agents according to claim 1, characterized in that they are suitable for oral application, resistant to gastric juice, and are released only in the mid and/or lower region of the small intestine.

3. Agents according to claim 2, characterized in that they contain said oxypurinol in the form of alkali and/or alkaline earth salts in amorphous or crystalline form, and are provided as tablets or capsules resistant to gastric juice.

4. Agents according to claim 2, characterized in that they contain said oxypurinol and/or its alkali, alkaline earth or ammonium salts in suspended or dissolved form, and are provided as capsules resistant to gastric juice.

5. Agents according to claim 1, characterized in that they contain said oxypurinol and/or its alkali, alkaline earth or ammonium salts in solid, suspended or dissolved form, and are provided in the form of clysmae.

6. Agents according to any of claims 1 to 4, characterized in that they contain 50 to 800 mg per dose unit.

7. Agents according to claim 5, characterized in that they contain 50 to 500 mg per dose unit.

8. Agents according to any of claims 1 to 7, characterized in that, in addition, they contain sulfasalazine or mesalazine.

9. Use of oxypurinol and/or its alkali, alkaline earth, or ammonium salts for the preparation of agents for enteral treatment of chronically inflammatory intestinal diseases.

10. Use of oxypurinol and/or its alkali, alkaline earth, or ammonium salts for the preparation of agents for enteral treatment of chronically inflammatory intestinal diseases, said agents containing a pharmacologically active dose of agent according to at least one of claims 1 to 8.

## Revendications

1. Médicaments pour le traitement entérique de maladies inflammatoires chroniques des instestins, contenant, en plus des excipients et adjuvants usuels, des doses pharmacologiquement actives d'oxypurinol et/ou de ses sels alcalins, alcalino-terreux ou d'ammonium, qui ne libèrent le principe actif que dans la région centrale et/ou inférieure de l'intestin grêle ou qu'on peut utiliser comme clystère.

2. Médicaments selon la revendication 1, caractérisés en ce qu'ils conviennent pour une administration orale, sont résistants au suc gastrique et ne sont libérés que dans la région centrale et/ou inférieure de l'intestin grêle.

3. Médicaments selon la revendication 2, caractérisés en ce qu'ils contiennent l'oxypurinol sous la forme de sels alcalins ou alcalino-terreux à l'état amorphe ou cristallin et se présentent sous la forme de comprimés ou de capsules résistants au suc gastrique.

4. Médicaments selon la revendication 2, caractérisés en ce qu'ils contiennent l'oxypurinol et/ou ses sels alcalins, alcalino-terreux ou d'ammonium en suspension ou dissous et se présentent sous la forme de capsules résistantes au suc gastrique.

5. Médicaments selon la revendication 1, caractérisés en ce qu'ils contiennent l'oxypurinol et/ou ses sels alcalins, alcalino-terreux ou d'ammonium à l'état solide, en suspension ou dissous et se présentent sous la forme de clystères.

6. Médicaments selon une des revendications 1 à 4, caractérisés en ce qu'ils contiennent 50 à 800 mg de principe actif par dose unitaire.

7. Médicaments selon la revendication 5, caractérisés en ce qu'ils contiennent 50 à 500 mg de principe actif par dose unitaire.

8. Médicaments selon une des revendications 1 à 7, caractérisés en ce qu'ils contiennent en outre de la sulfasalazine ou de la mésalazine.

9. Utilisation de l'oxypurinol et/ou de ses sels alcalins, alcalino-terreux ou d'ammonium pour la fabrication de médicaments pour le traitement entérique de maladies inflammatoires chroniques des intestins.

10. Utilisation de l'oxypurinol et/ou de ses sels alcalins, alcalino-terreux ou d'ammonium pour la fabrication de médicaments pour le traitement entérique de maladies inflammatoires chroniques des intestins, les médicaments contenant une dose pharmacologiquement active du médicament selon au moins une des revendications 1 à 8.
